# EUROPEAN PATENT SPECIFICATION

(11) **EP 3 033 016 B1**
(45) Date of publication and mention of the grant of the patent: **27.02.2019**
(21) Application number: 14755568.4
(22) Date of filing: 15.08.2014
(51) Int. Cl.: A61B 17/32, A61B 17/3207

(54) **SURGICAL INSTRUMENT**
CHIRURGISCHES INSTRUMENT
INSTRUMENT CHIRURGICAL

(30) Priority: 16.08.2013 US 201361866563 P
(43) Date of publication of application: 22.06.2016
(73) Proprietor: Covidien LP, Mansfield, MA 02048 (US)
(72) Inventor: AKILIAN, Mireille, Somerville, Massachusetts 02143 (US); CESARINI, Peter, Londondeery, New Hampshire 03053 (US)
(74) Representative: Maschio, Antonio
(86) International application number: PCT/US2014/051315
(87) International publication number: WO 2015/023965

(56) References cited:
- WO-A1-98/24373
- US-A- 4 031 765
- US-A1- 2004 092 980
- US-A1- 2012 172 889

## Description

### FIELD OF THE INVENTION

This disclosure generally relates to surgical instruments, and more particularly, to surgical instruments that include a cutting element.

### BACKGROUND

Conventional arthroscopic surgical instruments generally include an outer tube and an inner member that rotates or moves linearly within the outer tube. The outer tube and inner member may interact to create shear forces that cut tissue. This type of cutting is generally used to cut soft tissue, such as muscle, ligaments, and tendons.

US 2012/0172889 A1 discloses a tissue removal system including an inner tubular member configured to rotate and oscillate axially relative to an outer tubular member, and a reversible motor comprising a rotatable output shaft operatively coupled to the inner tubular member. A proximal end of the inner tubular member is disposed within, and configured to rotate and oscillate axially relative to, a housing. A first control switch disposed within the housing is actuated when the inner tubular member is in a distal position relative to the housing, and a second control switch disposed external to the housing is actuated by a user. The system further includes motor control circuitry configured such that when the second control switch is actuated, actuation of the first control switch causes the motor output shaft to change direction, and when the second control switch is not actuated, actuation of the first control switch causes the motor to turn off.

### BRIEF DESCRIPTION OF THE DRAWINGS

FIG. 1A is a partial top view of a surgical instrument according to some implementations of the present disclosure;
FIG. IB is a cross-sectional view taken along IB-IB in FIG. 1A;
FIG. 2A is a top view of an inner drive hub of the surgical instrument of FIG. 1;
FIG. 2B is a cross-sectional view taken along 2B-2B of the inner drive hub of FIG. 2A;
FIG. 2C is a distal end view of the inner drive hub of FIG. 2A;
FIG. 2D is a proximal end view of the inner drive hub of FIG. 2A;
FIG. 3A is a top view of a helical member of the surgical instrument of FIG. 1;
FIG. 3B is a side view of the helical member of FIG. 3 A;
FIG. 3C is a cross-sectional view taken along 3C-3C of the helical member of FIG. 3A;
FIG. 3D is a proximal end view of the helical member of FIG. 3 A;
FIG. 4A is a top view of an outer hub of the surgical instrument of FIG. 1;
FIG. 4B is a cross-sectional view taken along 4B-4B of the outer hub of FIG. 4A;
FIG. 4C is a distal end of the outer hub of FIG. 4A;
FIG. 5A is an exploded perspective view of a coupling piece and the helical member of the surgical instrument of FIG. 1;
FIG. 5B is an assembled partial cutaway view of the coupling piece and the helical member shown in FIG. 5A;
FIG. 5C is an assembled side view of the coupling piece and the helical member shown in FIG. 5A;
FIG. 5D is an assembled perspective view of coupling piece and the helical member shown in FIG. 5C;
FIG. 6A is a side view of a follower of the coupling piece of the surgical instrument of FIG. 1;
FIG. 6B is a cross-sectional view taken along 6B-6B of the follower of FIG. 6A;
FIG. 6C is a top view of the follower of FIG. 6A;
FIG. 7A is a top view of a cap of the follower of the coupling piece of the surgical instrument of FIG. 1;
FIG. 7B is a cross-sectional view taken along 7B-7B of the cap of FIG. 7A;
FIG. 8A is a partial top view of an outer member of the surgical instrument of FIG. 1;
FIG. 8B is a partial side view of the outer member of FIG. 8 A;
FIG. 9 is a partial side view of an inner member of the surgical instrument of FIG. 1;
FIG. 10 illustrates the surgical instrument of FIG. 1 in use to cut tissue;

FIG. 11 is a partial side view of an alternate implementation of an inner member of a surgical instrument according to some implementations of the present disclosure; and

FIG. 12 is a partial perspective view of an alternate implementation of a helical member of a surgical instrument according to some implementations of the present disclosure.

While the invention is susceptible to various modifications and alternative forms, a specific implementation thereof has been shown by way of example in the drawings and will herein be described in detail. It should be understood, however, that it is not intended to limit the invention to the particular forms disclosed, but on the contrary, the intention is to cover all modifications, equivalents, and alternatives falling within the scope of the invention as defined by appended claims.

### DESCRIPTION

As described above, surgical instruments include an outer tube and an inner member, where the inner member moves relative to the outer tube to create shear forces that are employed to cut tissue. According to aspects of the present invention, embodiments of surgical instruments are configured to achieve desired relative movement between the outer tube and the inner member. According to aspects of the present invention, the inner member moves linearly at different rates depending on its position and/or direction of movement relative to the outer tube. Thus, in such embodiments, the surgical instruments are configured to provide a back-and-forth linear motion that optimizes their cutting performance and/or other aspects of their operation.

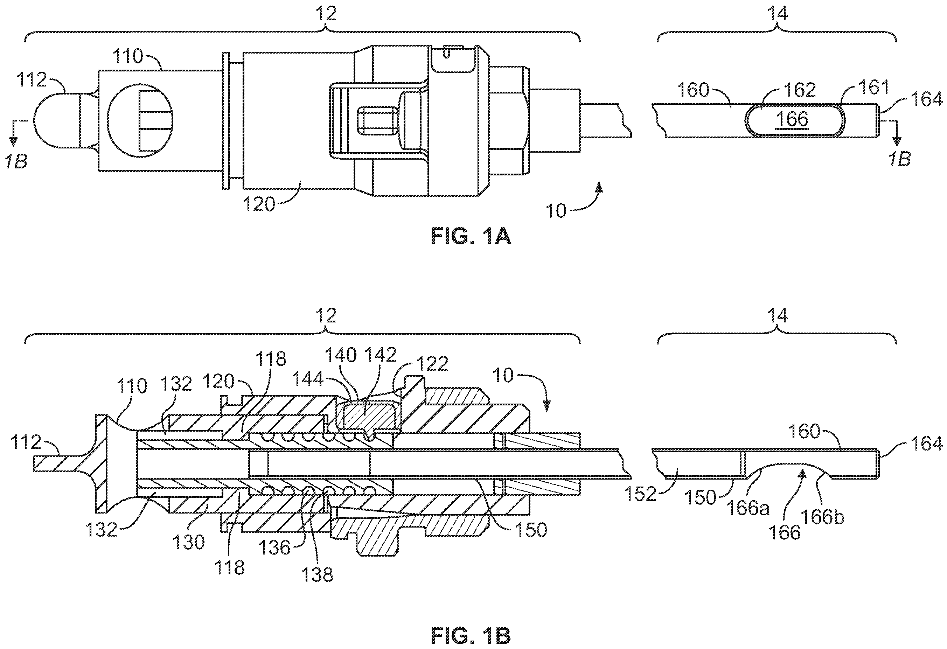

In some embodiments, the inner member is rotated relative to the outer tube and the rotation causes the inner member also to move linearly and back-and-forth relative to the outer tube. As an example, a cutting device 10 is described with reference to FIGS. 1-11. As shown in FIGS. 1A and 1B, the cutting device 10 includes a driving end 12 and a cutting end 14. The driving end 12 is located at the proximal end of the cutting device 10. The cutting end 14 is located at the distal end of the cutting device 10.

At the driving end 12, there is an inner drive hub 110 and an outer hub 120. The inner drive hub 110 includes a drive coupler 112, which mounts into a rotary driver (not shown). The rotary driver turns the drive coupler 112 causing a helical member or drive member 130 and the inner drive hub 110 to rotate. The helical member or drive member 130 is located within the inner drive hub 112 and the outer hub 120. The helical member or drive member 130 and a coupling piece 140 engage each other so that rotation of the helical member or drive member 130 causes linear motion of the helical member or drive member 130.

The cutting device 10 includes an elongated inner member or cutting member 150 and an elongated outer member 160, as shown in FIG. 1B. The inner member or cutting member 150 is tubular with a hollow interior 152 and used to cut or slice/shear tissue. The inner member 150 is coupled to the helical member 130 to enable linear and rotary motion of the inner member 150.

The outer member 160 is also tubular with a hollow interior 162. The inner member 150 is received inside the outer member 160. The outer member 160 is coupled to the outer hub 120. The outer member 160 may include a tip 164, which is blunt, e.g., the corners are rounded. At the cutting end 14, the outer member 160 defines a cutting window 166 through a wall 161 (FIG. 1A) of the outer member 160.

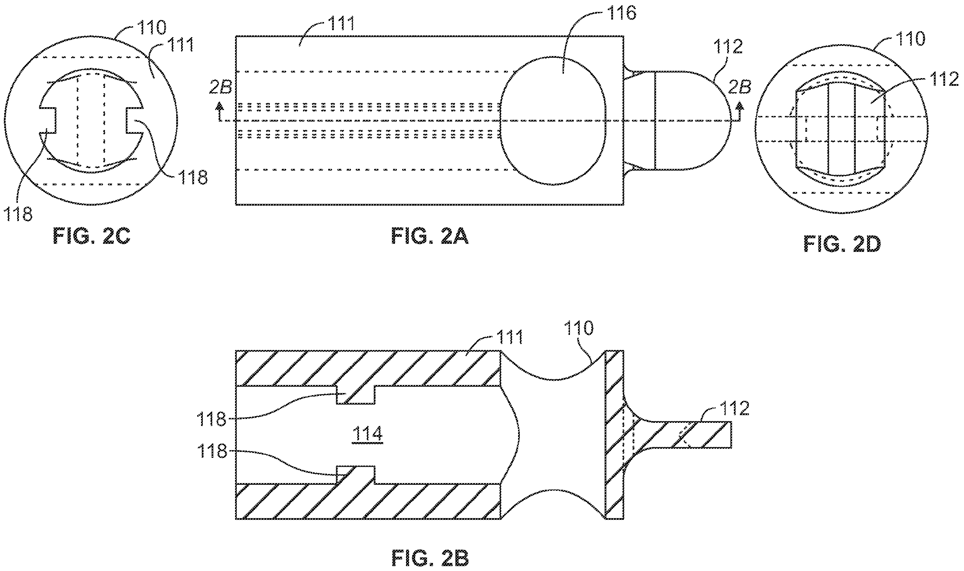

Referring to FIGS. 2A-2D, the inner drive hub 110 may include the drive coupler 112 (FIGS. 2A, 2B, 2D), a lumen 114 (FIG. 2B), an aspiration opening 116 (FIG. 2A), and at least one key 118 (FIGS. 2B and 2C). Debris from the cutting end 14 (FIGS. 1A and 1B) of the cutting device 10 may be aspirated through the aspiration opening 116. The drive coupler 112 extends from the proximal end of the inner drive hub 110 and couples the inner drive hub 110 to the rotary driver (not shown). The rotary driver may include a drive motor that is coupled to the drive coupler 112 to cause the drive hub 110 to rotate. The drive hub 110 transfers rotary motion to the helical member 130 while allowing the inner member 150 (which is coupled to the helical member) to move axially along the axis of rotation.

At least one key 118 extends from a wall 111 of the inner drive hub 110. Each key 118 functions as a guide along one side of the inner drive hub 110. Each key 118 of the inner drive hub 110 engages a respective slot 132 of the helical member 130 so that rotation of the inner drive hub 110 causes the helical member 130 to rotate while allowing the helical member 130 to move linearly relative to the inner drive hub 110, e.g., each key 118 slides linearly along the respective slot 132. As shown in FIGS. 1B and 2B-2D, the at least one key 118 is shaped like a fin and the at least one slot 132 is located at the proximal end of the helical member 130 to receive the at least one key 118 of the inner drive hub 110. In alternative implementations, the at least one slot is conversely disposed in the wall 111 of the inner drive hub 110 while the at least one key extends from a wall of the helical member 130 to engage the at least one slot. In the illustrated implementations, a pair of keys 118 engages respective slots 132. In general, however, any number of keys 118 may extend from inner drive hub 110 to engage respective slots 132 in the wall of the helical member 130, or vice versa. In alternative implementations, the rotary driver (not shown) may be coupled to the helical member 130 by gears or a gear and a spline gear.

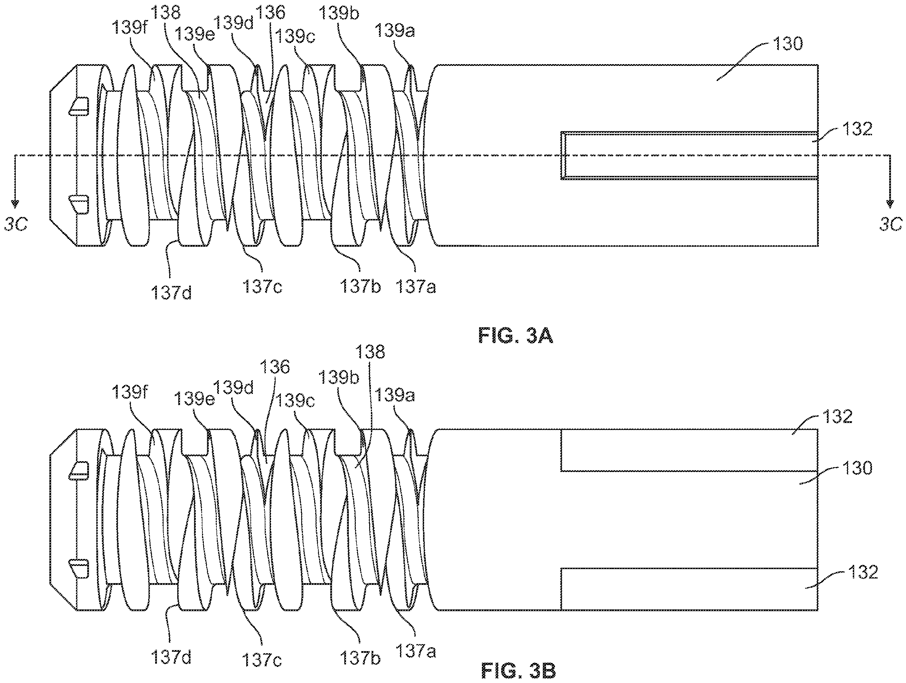

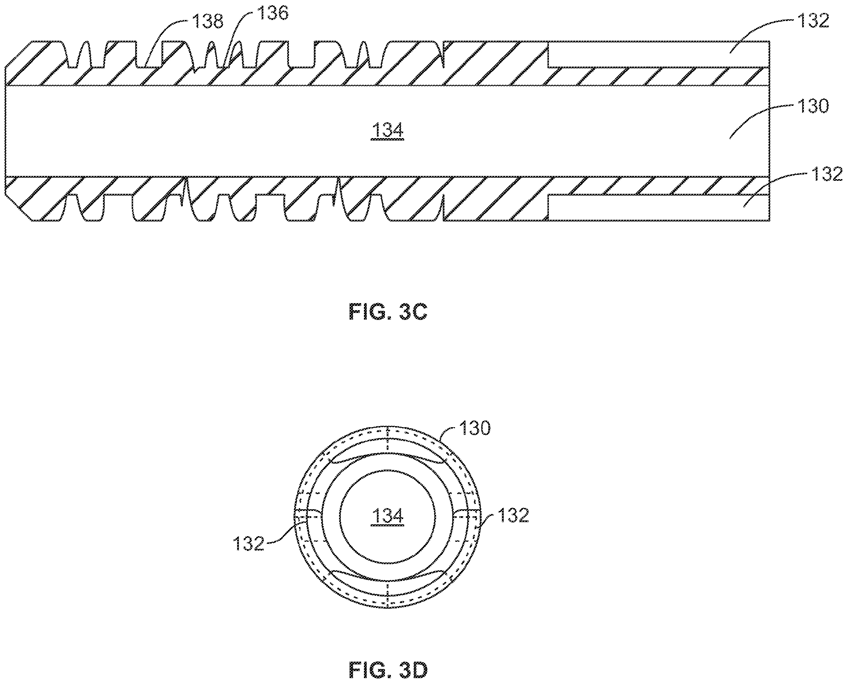

Referring to FIGS. 3A-3D, the helical member 130 of the cutting device 10 is formed of a material in a tubular shape with a lumen 134 (FIGS. 3C and 3D). The inner member 150 may be disposed within the lumen 134 of the helical member 130 and fixed therein, for example, by set screws, epoxy, injection-molded, or over-molded plastic. In alternative implementations, the inner member 150 may be coupled to the helical member 130 by a spline, gears or a gear and a spline.

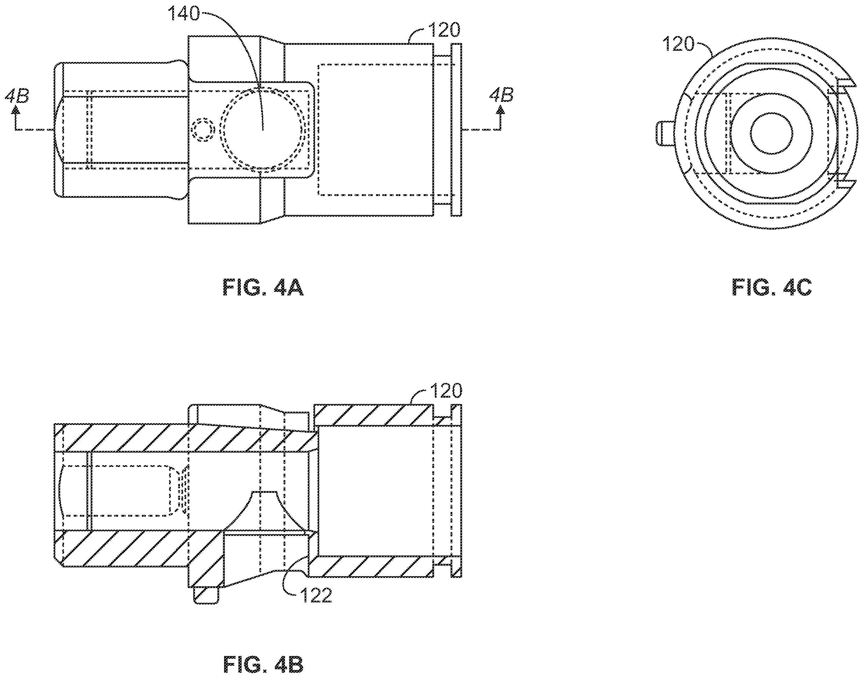

Referring to FIGS. 4A-4C, the outer hub 120 of the cutting device 10 is formed of hard plastic and does not move. A cutout 122 (FIG. 4B) is disposed within a wall of the outer hub 120, for example, centrally, as in FIG. 4B, and aligned with the helical member 130. As shown in FIG. 1B, the coupling piece 140 is located in the cutout 122 of the outer hub 120.

As shown in FIG. 1B, the outer member 160 is disposed within the outer hub 120 and fixed therein by a coupling using, for example, set screws, epoxy, glue, insert molding, or spin-welding.

Referring particularly to FIGS. 3A-3C, the helical member 130 also includes two helical channels 136, 138. The helical channels 136, 138 are disposed on a distal portion of the exterior surface of the helical member 130. As shown, the helical channel 136 is righthand threaded; the other helical channel 138 is left-hand threaded. The length of the distal portion of the helical member 130 with helical channels 136, 138 may be longer, shorter, or the same length as the length of the cutting window 166 (FIGS. 1A and 1B). The helical channels 136, 138 may be smoothly blended together at their ends to form a continuous groove so that there is a smooth transition from one helical channel to the other helical channel at each end of the distal portion of the helical member 130. The continuous groove provides for linear motion of the inner member 150 that includes moving distally over a length of travel and then changing direction and moving proximally over a length of travel and then changing direction to begin moving distally again. The length of travel can be determined as a function of the extent of the helical channels 136, 138 over the helical member 130. The velocity of the linear motion can be determined as a function of the angle or pitch of the helical channels 136, 138 and the rotational speed of the helical member 130. Changing direction includes, while moving in a first direction, decelerating to zero velocity and then accelerating in the opposite direction.

In accordance with some implementations of the present disclosure, the helical member 130 may be mechanically driven by the rotary driver (not shown) and moves linearly over a length of travel and then changes direction as a result of the interaction of the coupling piece 140 with the helical channels 136, 138. In such implementations, only a rotational force in a single rotational direction applied by the rotary driver to the helical member 130 is needed to drive the helical member 130. By drive the helical member 130 it is meant that the helical member is caused to rotate and move linearly in a back-and-forth motion. In accordance with other implementations of the present disclosure, the helical member 130 may be mechanically driven by the rotary driver and the coupling piece 140 moves linearly over a length of travel and then changes direction as a result of the interaction of the coupling piece 140 with the helical channels 136, 138. The coupling piece 140 can be coupled to the inner member 150 and cause the inner member 150 to move linearly and then change direction.

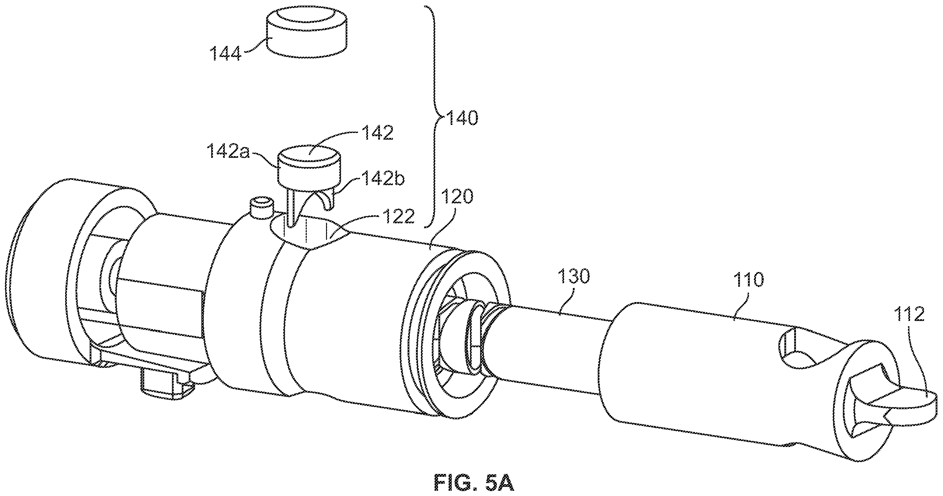

Referring to FIG. 5A, the coupling piece 140 includes a follower 142 and a cap 144. Having the two helical channels 136, 138 that are smoothly blended together at their ends to form a continuous groove in conjunction with the slot 132/key 118 coupling of the inner drive hub 110 and the helical member 130, the rotary driver only needs to rotate in a single direction and does not require reversal of the rotational direction upon the coupling piece 140 reaching the end of one of the helical channels 136, 138. That is, the helical member 130 is caused to move distally in a first direction and then in a second opposite direction without having to change the rotational direction of the rotary driver.

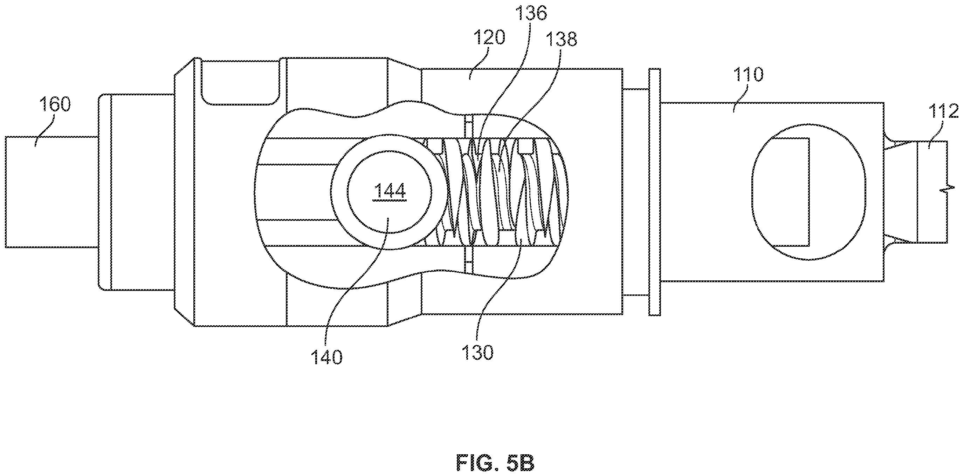

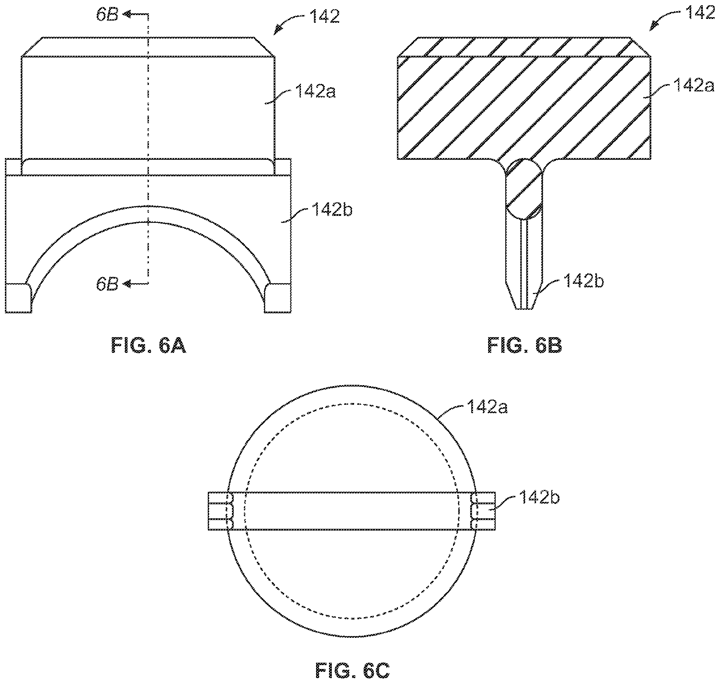

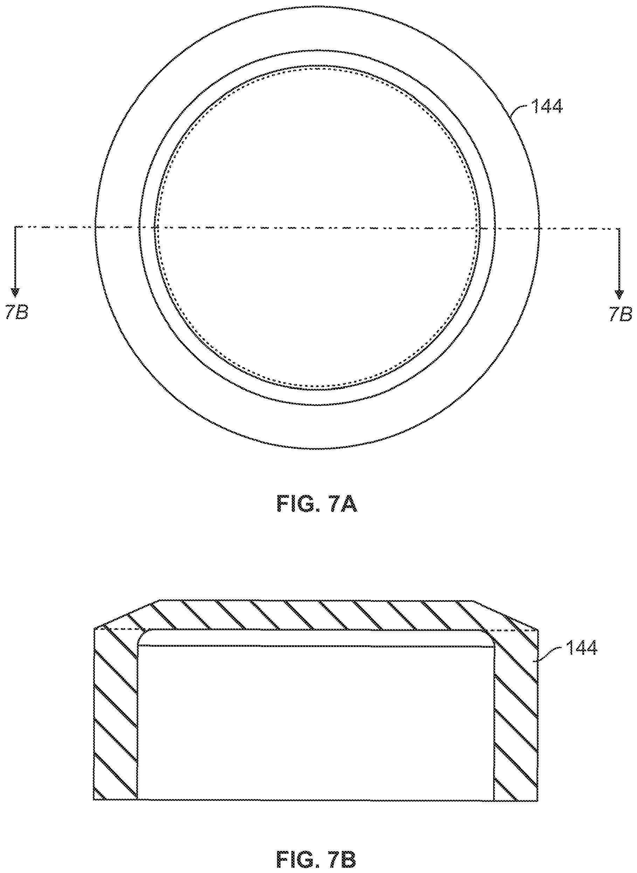

Referring to FIGS. 6A-6C, the follower 142 includes a cylindrical head 142a and two legs 142b. As shown in FIGS. 5B-5D, the legs 142b form an arch and rest in the channels of the helical channels 136, 138 formed in the distal portion of the exterior surface of the helical member 130. The arch of the legs 142b is dimensionally related to the diameter described by the helical channels 136, 138 of the helical member 130. Referring to FIGS. 7A and 7B, the cap 144 of the coupling piece 140 is shown, which, as best shown in the partially exploded view of FIG. 5A, covers the follower 142 to provide a seal to allow sufficient suction to remove aspirated debris. Also, the cap 144 may be a separate piece from the follower 142 in order to allow the follower 142 to swivel.

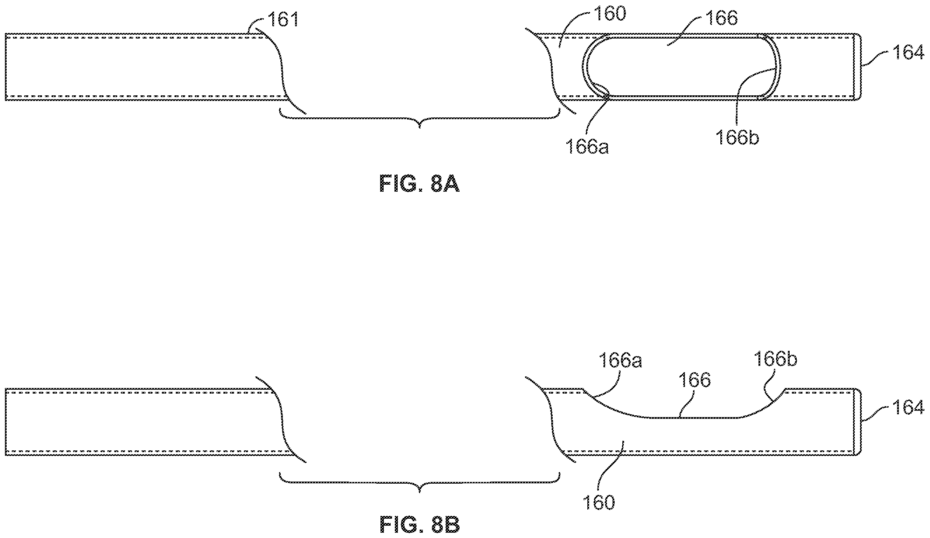

As shown in FIGS. 8A and 8B, the cutting window 166 has a generally oblong shape and is disposed proximate to the tip 164 of the outer member 160 and along the length of the outer member 160 from the distal tip 164 to a position proximate the helical member 130. The cutting window 166 exposes the inner member 150 over a length. The proximal end 166a of the cutting window 166 is U-shaped. The distal end 166b of the cutting window 166 is also U-shaped. It is understood, however, that the cutting window 166 may be shaped and/or positioned in a manner different from the illustrated embodiments. In some embodiments, the distal end 166b may optionally provide a sharp edge. In further embodiments, the distal end 166b may optionally have a saddle shape that forms a hook, which may pierce the targeted tissue to hold the tissue as the inner member 150 cuts.

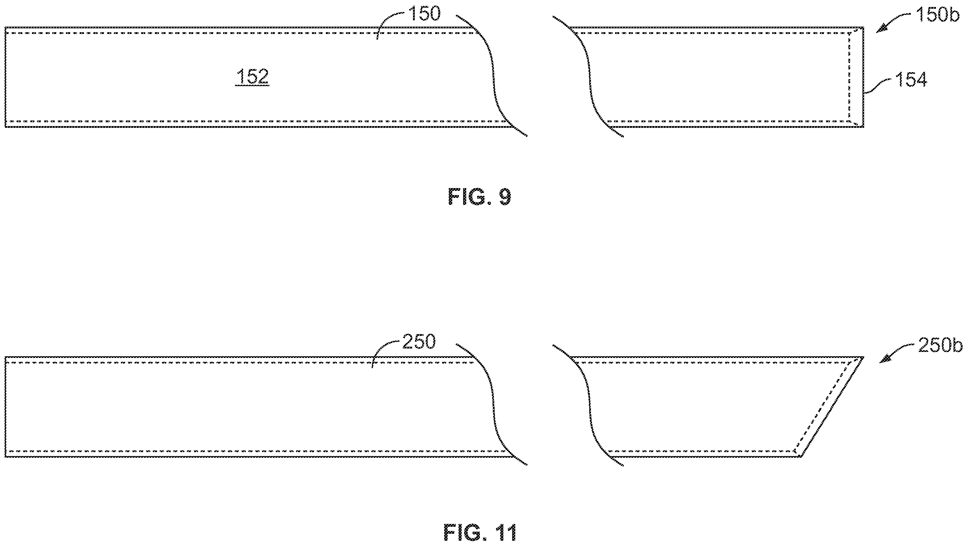

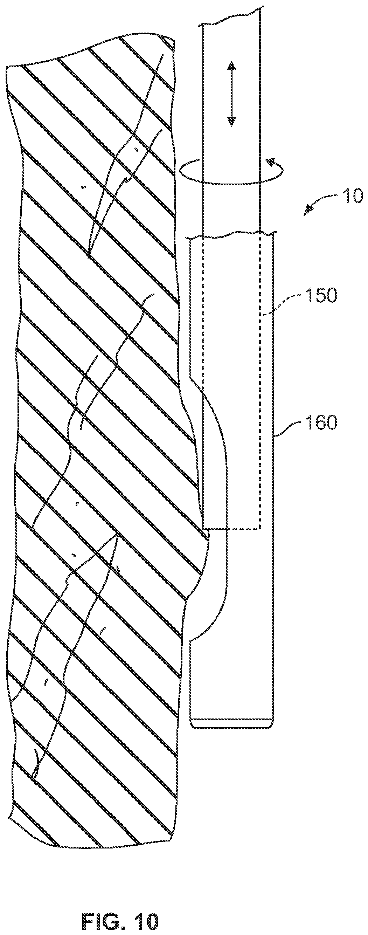

FIG. 9 shows that the inner member 150 is generally tubular with a hollow interior 152. Aspiration of debris occurs through the hollow interior 152 of the inner member 150, and through the lumen 134 (FIGS. 3C and 3D) of the helical member 130 to the aspiration opening 116 (FIG. 2A) of the inner drive hub 110. The distal end 150b of the inner member 150 is chamfered to a sharp edge 154 for cutting. The inner member 150 simultaneously rotates about its axis and moves linearly along its axis of rotation to cut tissue. The cutting surface of the distal end 150b of the inner member 150 shears tissue. For example, referring to FIG. 10, the cutting device 10 is placed tangentially against the targeted tissue such that the cutting window 166 exposes the inner member 150 to the tissue. The tissue protrudes through the cutting window 166 prior to being cut by the inner member 150. As the inner member 150 rotates and moves linearly (e.g., downward in the orientation shown in FIG. 10), as shown by the arrows, the cutting edge 154 of the inner member 150 shears the tissue as the inner member 150 advances to cut the tissue. The cut is completed as the cutting edge 154 (FIG. 9) of the inner member 150 advances beyond the distal end 166b (FIGS. 8A and 8B) of the cutting window 166 within the outer member 160.

FIG. 11 shows an alternative implementation of the inner member. The distal end 250b of the inner member 250 may be angled to a chamfered point so that the cut in the targeted tissue is initiated on one side and then extends across the width of the tissue. Similarly, when the cutting device is placed tangentially against the targeted tissue, the rotating and linearly moving inner member 250 shears the tissue to be cut.

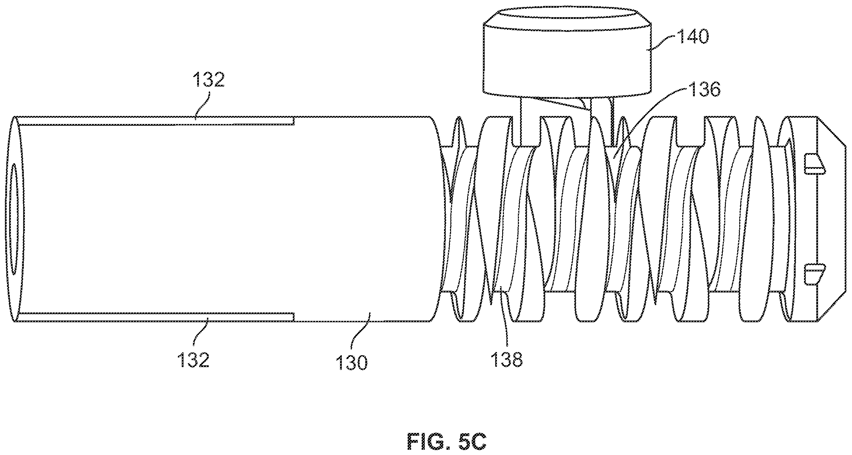

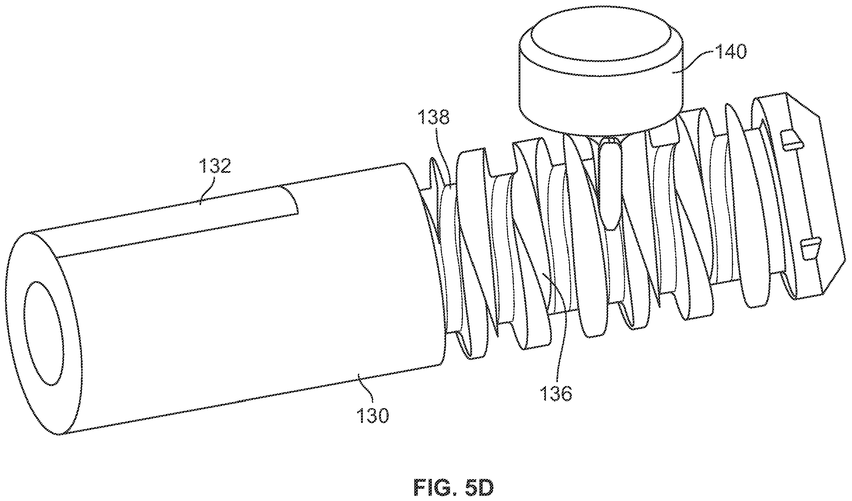

Referring particularly to FIGS. 5C and 5D, as the helical member 130 and the inner drive hub 110 (FIGS. 2A-2D) are mechanically driven by the rotary driver (not shown), the follower 142 (FIGS. 6A and 6B) of the coupling piece 140 follows the helical channels 136, 138, swiveling as the follower 142 smoothly transitions from helical channel 136 to helical channel 138 at the ends of the distal portion of the helical member 130 having the helical channels 136, 138. The coupling of the follower 142 to the helical channels 136, 138 causes the helical member 130 to also move linearly. Thus, the inner member 150 simultaneously rotates and moves linearly to cut the tissue.

When the helical member 130 moves toward the distal end, the cutting edge 154 (FIG. 9) of the inner member 150 advances and closes the cutting window 166 (FIGS. 8A and 8B) so that the cutting device 10 engages and cuts targeted tissue. Meanwhile, when the helical member 130 moves away from the distal end, the cutting edge 154 withdraws and opens the cutting window 166 so that the resulting debris can be aspirated through the window 166 and into the hollow interior 152 of the inner member 150. In addition, the opening of the cutting window 166 allows tissue to be drawn in for the next cut. Because the cutting device 10 performs different operations (e.g., cutting, aspirating, etc.) when the helical member 130 moves toward or away from the distal end, it is advantageous to optimize the movement of the helical member 130 according to the different functions. It is advantageous to move the helical member 130 more slowly away from the distal end and thus keep the cutting window 166 open for a relatively longer period of time after a cut to allow sufficient aspiration of debris and to provide time for more tissue to enter the cutting window 166 in preparation for the next cut. Accordingly, aspects of the present disclosure provide a helical device that is configured to move linearly at different rates depending on its position and/or direction of movement.

The legs 142b (FIG. 6A) of the follower 142 of the coupling piece 140 travel over the helical channels 136, 138 to produce the desired linear motion of the helical member 130 from the input rotary motion. Movement of the coupling piece 140 along the first helical channel 136 causes the helical member 130 to move toward the distal end and causes the cutting device to perform the cutting operation. Meanwhile, movement of the coupling piece 140 along the second helical channel 138 causes the helical member 130 to move away from the distal end and allows the cutting device to perform the aspirating operation and draw more tissue into the cutting window 166 for cutting.

The first helical channel 136 is defined by a thread with a first helical angle or pitch. As shown in FIGS. 3A-3B, the thread for the first helical channel 136 defines four turns 137a-d distributed evenly along a distance of the helical member 130. Meanwhile, the second helical channel 138 is defined by a thread with a second helical angle or pitch. The thread for the second helical channel 138 defines six turns 139a-f distributed evenly along the same distance of the helical member 130. Thus, the first helical channel 136 has fewer turns than the second helical channel 138 over the same distance. The number of turns over a distance generally corresponds to the number of rotations required by the helical member 130 to travel linearly over the same distance. To define more turns over a given distance of the helical member 130, a thread must generally have a smaller helical angle or pitch. Thus, the second helical angle or pitch associated with the second helical channel 138 is smaller than the first helical angle or pitch associated with the first helical channel 136.

To move linearly over a given distance, the helical member 130 must make fewer rotations when the coupling piece 140 travels over the first helical channel 136, i.e., when the helical member 130 moves toward the distal end. Conversely, the helical member 130 must make more rotations when the coupling piece 140 travels over the second helical channel 138, i.e., when the helical member 130 moves away from the distal end. When the helical member 130 is rotated at a constant speed, (1) the helical member 130 moves at a relatively faster linear speed when it is moving toward the distal end to perform the cutting operation and (2) conversely, the helical member 130 moves at a relatively slower linear speed when the helical member 130 is moving away from the distal end to perform the aspirating operation and draw more tissue into the cutting window 166 for subsequent cutting.

The first helical channel 136 may be configured with a particular helical angle or pitch so that the cutting device performs the cutting operation at a particular linear speed for optimal performance. Meanwhile, the second helical channel 138 is configured with a relatively smaller helical angle or pitch to keep the cutting window 166 at least partially open for a longer time. As described above, it is advantageous to move the helical member 130 more slowly away from the distal end and thus keep the cutting window 166 open for a longer period of time after a cut. This allows for sufficient aspiration of debris and provides time for more tissue to enter the cutting window 166 in preparation for the next cut.

Although the thread for the first helical channel 136 defines four turns 137a-d and the thread for the second helical channel 138 defines six turns 139a-f over the same linear distance of the helical member 130, it is understood that, in other embodiments, the first helical channel and the second helical channel may be configured with different respective helical angles so they have different respective numbers of turns than shown in FIGS. 3A-3C. In addition, although the entire length of the first helical channel 136 may be defined by the first helical angle or pitch and the entire length of the second helical channel 138 may be defined by the second helical angle or pitch in FIGS. 3A-3C, it is understood that other implementations may employ one or more helical channels that are defined by multiple helical angles or pitches that cause the helical member to move at various different speeds as the helical member moves in one direction. Furthermore, it is understood that in examples not part of the present invention, the helical channels may be configured so that the first helical channel has a smaller helical angle than the helical angle of the second helical channel, thereby causing the helical member to move relatively slower toward the distal end (to perform the cutting operation more slowly) and relatively faster away from the distal end (to perform the aspirating operation, etc. more quickly).

The helical member 130 can be generally used in the cutting device 10 described above. It is understood, however, that aspects of the helical member 130 may be employed in other types of cutting devices to achieve corresponding advantages.

In general, according to some aspects of the present disclosure, surgical instruments employ a helical member with helical channels that are configured to provide optimal linear motion. The helical channels can be smoothly blended at their ends to provide a continuous channel that provides for a change in direction at the ends of the linear motion. In particular, the helical channels are defined by threads with different helical angles so that the rotation of the helical member causes linear movement at different desired linear speeds.

The resulting linear movement may involve relative movement between any components of the surgical instruments and are not limited to the examples and implementations described herein. The helical member may be fixed to a first component (e.g., in a housing) and rotation of the helical member relative to a second component also causes relative linear movement between the first and second components. In accordance with some implementations of the present disclosure, the helical member can be stationary and a follower can be permitted to move linearly along the helical member, such as in a carriage or guide. The follower can be coupled to the inner member to cause the inner member to move linearly as the follower is moved by rotational motion of the helical member. A separate drive train, such as gears, belts and pulleys, can be used to impart rotary motion on the inner member. In accordance with some implementations of the present disclosure, the inner member can rotate about the same axis, a parallel axis, or a non-parallel axis as the helical member.

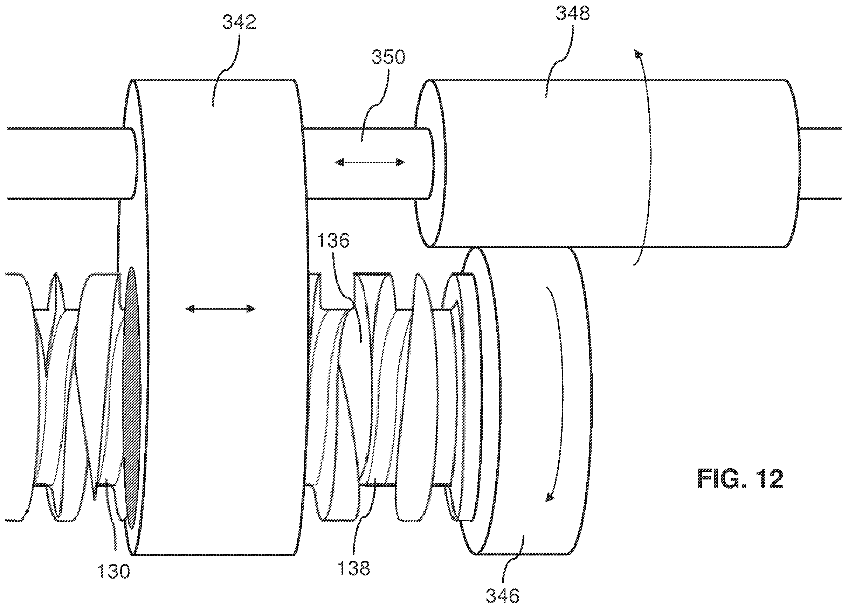

For example, the helical member 130 shown in FIG. 12 is driven by a rotary driver (not shown), but unlike the helical member 130 in the implementations of FIGS. 1A-11, the helical member 130 in FIG. 12 does not move linearly together with inner member 350. Rather, the inner member 350 moves linearly relative to the helical member 130. For example, the helical member 130 may remain stationary relative to a housing element while the inner member 350 moves linearly relative to the housing element. As FIG. 12 shows, a follower 342 moves linearly along the helical channels 136, 138 of the helical member 130. The movement of the follower 342 is determined by the helical channels 136, 138 as described above. The follower 342 is coupled to the inner member 350, so that the inner member 350 also moves linearly along a parallel axis according to the helical channels 136, 138. In particular, the inner member 350 moves linearly at different rates depending on its position and/or direction of movement relative to an outer member (not shown). To cause rotation of the inner member 350, the helical member 130 is coupled to a gear 346 that engages a gear 348 coupled to the inner member 350. The gear 346 slides along, and remains engaged with, the longer gear 348 as the gear 348 moves linearly with the inner member 350. Rotation of the helical member 130 causes corresponding rotation of the gears 346, 348 to rotate the inner member 350. Accordingly, the helical member 130 causes linear and rotary movement of the inner member 350 relative to the outer member to produce the desired cutting operation, aspirating operation, etc.

While the present disclosure has been described with reference to one or more particular implementations, those skilled in the art will recognize that many changes may be made thereto without departing from the scope of the present disclosure. Each of these implementations and obvious variations thereof is contemplated as falling within the scope of the present disclosure, wherein the scope of the invention is set forth in the following claims. It is also contemplated that additional implementations according to aspects of the present disclosure may combine any number of features from any of the implementations described herein.

## Claims

1. A surgical instrument, comprising:
an outer member (160) defining a cutting window (166);
a cutting member (150) at least partially positioned within the outer member, the cutting member including a sharp edge for cutting tissue protruding through the cutting window; and
a drive member (130) coupled to the cutting member such that in response to only a rotational force applied to the drive member in a single rotational direction, the cutting member (i) rotates and (ii) moves linearly in a back-and-forth motion, the drive member having a continuous helical groove including a first helical channel (136) and a second helical channel (138), the first helical channel and the second helical channel being blended at their respective ends to form the continuous helical groove, at least a portion of the first helical channel having a first helical angle that causes the cutting member to move linearly in a first direction at a first linear speed and at least a portion of the second helical channel having a second helical angle that is smaller than the first helical angle that causes the cutting member to move linearly in a second opposing direction at a second linear speed that is slower than the first linear speed when the drive member is rotated at a constant speed,
wherein the surgical instrument is configured to perform a cutting operation when the cutting member moves linearly in the first direction at the first linear speed and wherein the surgical instrument is configured to perform an aspirating operation when the cutting member moves linearly in the second direction at the second linear speed, and
wherein when the cutting member moves linearly in the first direction, the cutting member moves across the cutting window to close the cutting window and to cut tissue in the cutting window, and when the cutting member moves linearly in the second direction, the cutting member withdraws to open the cutting window.

2. The surgical instrument of claim 1, wherein during the aspirating operation, tissue and fluid is sucked through the cutting window, through the cutting member, and through a lumen in the drive member.

3. The surgical instrument of claim 1 or 2, wherein the at least a portion of the first helical channel defines a first number of evenly distributed turns (137a-137d) along a distance of the helical member and the at least a portion of the second helical channel defines a second number of evenly distributed turns (139a-139f) along the same distance of the helical member, the second number being greater than the first number.

4. The surgical instrument of any one of claims 1 to 3, wherein the first helical angle is a non-zero angle with respect to an axis of rotation of the drive member and wherein the second helical angle is a non-zero angle with respect to the axis of rotation of the drive member.

5. The surgical instrument of claim 4, wherein the first direction is parallel with the axis of rotation and wherein the second direction is parallel with the axis of rotation

6. The surgical instrument of any one of the preceding claims, further comprising a coupling piece (140) at least partially disposed in the continuous helical groove of the drive member such that rotation of the drive member causes the cutting member to move linearly in the back-and- forth motion.

7. The surgical instrument of claim 6, wherein in response to at least a portion of the coupling piece being disposed in the first helical channel, the cutting member being configured to move linearly in the first direction at the first linear speed and in response to the at least a portion of the coupling piece being disposed in the second helical channel, the cutting member being configured to move linearly in the second direction at the second linear speed.

8. The surgical instrument of claim 7, wherein the coupling piece includes a follower (142) having a head (142a) and two legs (142b), the at least a portion of the coupling piece being the two legs.

9. The surgical instrument of any one of the preceding claims, wherein the first helical channel is a right- hand threaded channel and the second helical channel is a left-hand threaded channel.

10. The surgical instrument of any one of the preceding claims, wherein the cutting member is tubular with a hollow interior.

11. The surgical instrument of any one of the preceding claims, wherein the outer member is tubular with a hollow interior, and wherein the outer member has a blunt tip adjacent to the cutting window.

12. The surgical instrument of any one of the preceding claims, wherein the cutting member rotates about an axis of rotation and moves linearly along the axis of rotation.

## Patentansprüche

1. Chirurgisches Instrument, umfassend:
ein äußeres Element (160), das ein Schneidfenster (166) definiert;
ein Schneidelement (150), das mindestens teilweise innerhalb des äußeren Elements positioniert ist, wobei das Schneidelement eine scharfe Kante zum Schneiden von Gewebe aufweist, das durch das Schneidfenster ragt; und
ein Antriebselement (130), das mit dem Schneidelement derart gekoppelt ist, dass als Reaktion auf nur eine auf das Antriebselement in einer einzigen Rotationsrichtung ausgeübte Rotationskraft, das Schneidelement (i) sich dreht und (ii) sich linear in einer Hin- und Herbewegung bewegt, wobei das Antriebselement eine kontinuierliche spiralförmige Rille aufweist, die einen ersten spiralförmigen Kanal (136) und einen zweiten spiralförmigen Kanal (138) aufweist, wobei der erste spiralförmige Kanal und der zweite spiralförmige Kanal an ihren jeweiligen Enden zum Bilden der kontinuierlichen spiralförmigen Rille verschmelzen, wobei mindestens ein Abschnitt des ersten spiralförmigen Kanals, der einen ersten Steigungswinkel aufweist, der bewirkt, dass sich das Schneidelement linear in eine erste Richtung mit einer ersten linearen Geschwindigkeit bewegt, und mindestens ein Abschnitt des zweiten spiralförmigen Kanals, der einen zweiten Steigungswinkel aufweist, der kleiner als der erste Steigungswinkel ist, der bewirkt, dass sich das Schneidelement linear in eine zweite entgegengesetzte Richtung mit einer zweiten linearen Geschwindigkeit bewegt, die langsamer als die erste lineare Geschwindigkeit ist, wenn das Antriebselement mit einer konstanten Geschwindigkeit gedreht wird,
wobei das chirurgische Instrument konfiguriert ist, um einen Schneidvorgang durchzuführen, wenn sich das Schneidelement linear in die erste Richtung mit der ersten linearen Geschwindigkeit bewegt, und wobei das chirurgische Instrument konfiguriert ist, um einen Ansaugvorgang durchzuführen, wenn sich das Schneidelement linear in die zweite Richtung mit der zweiten linearen Geschwindigkeit bewegt, und
wobei, wenn sich das Schneidelement linear in die erste Richtung bewegt, sich das Schneidelement über das Schneidfenster bewegt, um das Schneidfenster zu schließen und Gewebe in dem Schneidfenster zu schneiden, und wenn sich das Schneidelement linear in die zweite Richtung bewegt, sich das Schneidelement zurückzieht, um das Schneidfenster zu öffnen.

2. Chirurgisches Instrument nach Anspruch 1, wobei während des Ansaugvorgangs Gewebe und Fluid durch das Schneidfenster, durch das Schneidelement und durch ein Lumen in dem Antriebselement angesaugt wird.

3. Chirurgisches Instrument nach Anspruch 1 oder 2, wobei der mindestens eine Abschnitt des ersten spiralförmigen Kanals eine erste Anzahl von gleichmäßig verteilten Windungen (137a bis 137d) entlang einer Distanz des spiralförmigen Elements und der mindestens eine Abschnitt des zweiten spiralförmigen Kanals eine zweite Anzahl von gleichmäßig verteilten Windungen (139a bis 139f) entlang der gleichen Distanz des spiralförmigen Elements definiert, wobei die zweite Zahl größer als die erste Zahl ist.

4. Chirurgisches Instrument nach einem der Ansprüche 1 bis 3, wobei der erste Steigungswinkel ein Winkel ungleich Null in Bezug auf eine Rotationsachse des Antriebselements ist und wobei der zweite Steigungswinkel ein Winkel ungleich Null in Bezug auf die Rotationsachse des Antriebselements ist.

5. Chirurgisches Instrument nach Anspruch 4, wobei die erste Richtung parallel zur Rotationsachse ist und wobei die zweite Richtung parallel zur Rotationsachse ist.

6. Chirurgisches Instrument nach einem der vorhergehenden Ansprüche, weiter umfassend ein Kupplungsstück (140), das mindestens teilweise in der kontinuierlichen spiralförmigen Rille des Antriebselements angeordnet ist, sodass die Rotation des Antriebselements bewirkt, dass sich das Schneidelement in der Hin- und Herbewegung linear bewegt.

7. Chirurgisches Instrument nach Anspruch 6, wobei als Reaktion auf mindestens einen Abschnitt des Kupplungsstücks, der in dem ersten spiralförmigen Kanal angeordnet ist, das Schneidelement konfiguriert ist, um sich linear in der ersten Richtung mit der ersten linearen Geschwindigkeit zu bewegen und als Reaktion auf den mindestens einen Abschnitt des Kupplungsstücks, der in dem zweiten spiralförmigen Kanal angeordnet ist, das Schneidelement konfiguriert ist, um sich linear in der zweiten Richtung mit der zweiten linearen Geschwindigkeit zu bewegen.

8. Chirurgisches Instrument nach Anspruch 7, wobei das Kupplungsstück einen Folger (142) mit einem Kopf (142a) und zwei Schenkeln (142b) aufweist, wobei der mindestens eine Abschnitt des Kupplungsstücks die beiden Schenkel sind.

9. Chirurgisches Instrument nach einem der vorhergehenden Ansprüche, wobei der erste spiralförmige Kanal ein rechtsgängiger Gewindekanal und der zweite spiralförmige Kanal ein linksgängiger Gewindekanal ist.

10. Chirurgisches Instrument nach einem der vorhergehenden Ansprüche, wobei das Schneidelement rohrförmig mit einem hohlen Innenraum ist.

11. Chirurgisches Instrument nach einem der vorhergehenden Ansprüche, wobei das äußere Element rohrförmig mit einem hohlen Innenraum ist und wobei das äußere Element eine stumpfe Spitze angrenzend an das Schneidfenster aufweist.

12. Chirurgisches Instrument nach einem der vorhergehenden Ansprüche, wobei sich das Schneidelement um eine Rotationsachse dreht und sich linear entlang der Rotationsachse bewegt.

## Revendications

1. Instrument chirurgical, comprenant :
un élément extérieur (160) définissant une fenêtre de découpe (166) ;
un élément de découpe (150) positionné au moins en partie à l'intérieur de l'élément extérieur, l'élément de découpe incluant un bord tranchant pour la découpe de tissu faisant saillie à travers la fenêtre de découpe ; et
un élément d'entraînement (130) couplé à l'élément de découpe de sorte qu'en réponse à seulement à une force de rotation appliquée à l'élément d'entraînement dans une seule direction de rotation, l'élément de découpe (i) tourne et (ii) se déplace linéairement dans un mouvement de va-et-vient, l'élément d'entraînement ayant une rainure hélicoïdale continue incluant un premier canal hélicoïdal (136) et un second canal hélicoïdal (138), le premier canal hélicoïdal et le seconde canal hélicoïdal étant combinés à leurs extrémités respectives pour former la rainure hélicoïdale continue, au moins une partie du premier canal hélicoïdal ayant un premier angle hélicoïdal qui amène l'élément de découpe à se déplacer linéairement dans une première direction à une première vitesse linéaire et au moins une partie du second canal hélicoïdal ayant un second angle hélicoïdal qui est plus petit que le premier angle hélicoïdal qui amène l'élément de découpe à se déplacer linéairement dans une seconde direction opposée à une seconde vitesse linéaire qui est plus faible que la première vitesse linéaire lorsque l'élément d'entraînement est tourné à une vitesse constante,
dans lequel l'instrument chirurgical est configuré pour réaliser une opération de découpe lorsque l'élément de découpe se déplace linéairement dans la première direction à la première vitesse linéaire et dans lequel l'instrument chirurgical est configuré pour réaliser une opération d'aspiration lorsque l'élément de découpe se déplace linéairement dans la seconde direction à la seconde vitesse linéaire, et
dans lequel lorsque l'élément de découpe se déplace linéairement dans la première direction, l'élément de découpe se déplace à travers la fenêtre de découpe pour fermer la fenêtre de découpe et pour découper un tissu dans la fenêtre de découpe, et lorsque l'élément de découpe se déplace linéairement dans la seconde direction, l'élément de découpe se retire pour ouvrir la fenêtre de découpe.

2. Instrument chirurgical selon la revendication 1, dans lequel pendant l'opération d'aspiration, du tissu et du fluide sont aspirés à travers la fenêtre de découpe, à travers l'élément de découpe, et à travers une lumière dans l'élément d'entraînement.

3. Instrument chirurgical selon la revendication 1 ou 2, dans lequel l'au moins une partie du premier canal hélicoïdal définit un premier nombre de pas répartis uniformément (137a-137d) le long d'une distance de l'élément hélicoïdal et l'au moins une partie du second canal hélicoïdal définit un second nombre de pas répartis uniformément (139a-139f) le long de la même distance de l'élément hélicoïdal, le second nombre étant plus grand que le premier nombre.

4. Instrument chirurgical selon l'une quelconque des revendications 1 à 3, dans lequel le premier angle hélicoïdal est un angle non nul par rapport à un axe de rotation de l'élément d'entraînement et dans lequel le second angle hélicoïdal est un angle non nul par rapport à l'axe de rotation de l'élément d'entraînement.

5. Instrument chirurgical selon la revendication 4, dans lequel la première direction est parallèle à l'axe de rotation et dans lequel la seconde direction est parallèle à l'axe de rotation.

6. Instrument chirurgical selon l'une quelconque des revendications précédentes, comprenant en outre une pièce de couplage (140) disposée au moins en partie dans la rainure hélicoïdale continue de l'élément d'entraînement de sorte qu'une rotation de l'élément d'entraînement amène l'élément de découpe à se déplacer linéairement dans un mouvement de va-et-vient.

7. Instrument chirurgical selon la revendication 6, dans lequel en réponse au fait qu'au moins une partie de la pièce de couplage est disposée dans le premier canal hélicoïdal, l'élément de découpe est configuré pour se déplacer linéairement dans la première direction à la première vitesse linéaire et en réponse au fait qu'au moins une partie de la pièce de couplage est disposée dans le second canal hélicoïdal, l'élément de découpe est configuré pour se déplacer linéairement dans la seconde direction à la seconde vitesse linéaire.

8. Instrument chirurgical selon la revendication 7, dans lequel la pièce de couplage inclut un suiveur (142) ayant une tête (142a) et deux jambes (142b), l'au moins une partie de la pièce de couplage étant les deux jambes.

9. Instrument chirurgical selon l'une quelconque des revendications précédentes, dans lequel le premier canal hélicoïdal est un canal à filetage à droite et le seconde canal hélicoïdal est un canal à filetage à gauche.

10. Instrument chirurgical selon l'une quelconque des revendications précédentes, dans lequel l'élément de découpe est tubulaire avec un intérieur creux.

11. Instrument chirurgical selon l'une quelconque des revendications précédentes, dans lequel l'élément extérieur est tubulaire avec un intérieur creux, et dans lequel l'élément extérieur a une pointe émoussée adjacente à la fenêtre de découpe.

12. Instrument chirurgical selon l'une quelconque des revendications précédentes, dans lequel l'élément de découpe tourne autour d'un axe de rotation et se déplace linéairement le long de l'axe de rotation.
